# DEMANDE DE BREVET EUROPEEN

(11) **EP 4 372 411 A1**
(43) Date de publication de la demande: **22.05.2024**
(21) Numéro de dépôt: 23197811.5
(22) Date de dépôt: 15.09.2023
(51) Int. Cl.: G01S 7/52, G01S 15/89

(54) **PROCÉDÉ POUR PILOTER UN DISPOSITIF ÉMETTEUR ET/OU RÉCEPTEUR D'ONDES**

(30) Priorité: 18.11.2022 FR 2212005
(71) Demandeur: SUPERSONIC IMAGINE, 13290 Aix-en-Provence (FR)
(72) Inventeur: Cordonnier, Hubert, 13290 Aix-en-Provence (FR); Roux, Guillaume, 13290 Aix-en-Provence (FR); Martin, Jean-Alain, 13290 Aix-en-Provence (FR)
(74) Mandataire: RVDB Nantes

(57) **Abrégé**

**Procédé pour piloter un dispositif émetteur et/ou récepteur d'ondes**

La présente divulgation concerne un procédé et un système (10) pour piloter un premier dispositif (20) émetteur et/ou récepteur d'ondes (W1, W2). Le procédé comprend : pilotage du premier dispositif (20) au travers de canaux (CN) établis via des liaisons électriques (LN), dans lequel au moins deux desdits canaux (CN) sont permutés au cours du temps parmi les liaisons électriques (LN).

## Description

### Technique antérieure

Un dispositif émetteur et/ou récepteur d'ondes, tel qu'un dispositif transducteur, comprend habituellement une pluralité d'éléments transducteurs (par exemple disposés en réseau) à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine. Un exemple comprend l'imagerie par ultrasons.

A cet effet, le dispositif émetteur et/ou récepteur d'ondes peut être piloté au moyen de signaux électriques, ces signaux étant par exemple transmis entre le dispositif émetteur et/ou récepteur d'ondes et une unité de commande. Ainsi, des signaux électriques représentant des ondes peuvent être transmis à des éléments transducteurs du dispositif émetteur et/ou récepteur d'ondes, causant l'émission d'ondes dans un milieu donné. Des signaux électriques peuvent éventuellement être récupérés en retour, ces signaux représentant une réponse (ou écho) du milieu aux sollicitations ondulatoires.

Par exemple, l'imagerie ultrasonore peut avoir pour but d'estimer la réflectivité d'un milieu. La fréquence des signaux peut alors être choisie en fonction des caractéristiques du milieu observé (par exemple un tissu humain).

Plus particulièrement, dans un procédé conventionnel d'imagerie par ultrasons, on peut utiliser par exemple un dispositif transducteur d'ultrasons (également appelé sonde à ultrasons) équipé d'au moins un ou d'un ensemble d'éléments transducteurs d'ultrasons. Dans un tel procédé, un ou plusieurs transducteurs sont utilisés pour convertir les signaux électriques en ondes ultrasonores. En particulier, les transducteurs peuvent émettre un ou successivement plusieurs faisceaux ultrasonores en direction d'un milieu, ce qui correspond à une opération d'émission. Ensuite, dans une opération de réception, un ensemble de signaux d'écho rétrodiffusés sont reçus du milieu par le même ensemble ou par un autre ensemble d'éléments transducteurs. En particulier, chacun des éléments transducteurs peut par exemple convertir un signal d'écho reçu en un signal électrique. Le signal peut ensuite être traité par le système à ultrasons ou par tout système associé (dédié), directement connecté ou non. Par exemple, le signal peut être amplifié, filtré, numérisé et/ou une opération de conditionnement du signal peut être effectuée. Les éléments transducteurs peuvent être disposés selon une ligne de transducteurs, une matrice, ou comme un réseau de transducteurs ou toute autre configuration.

Cependant, le pilotage des dispositifs émetteurs et/ou récepteurs d'ondes, tels que les dispositifs transducteurs par exemple, ne permet pas toujours d'obtenir des signaux électriques de façon satisfaisante. Il a été constaté que les signaux électriques transmis aux, et reçus depuis, les dispositifs émetteur et/ou récepteur d'ondes sont parfois perturbés ou ne sont pas optimaux, ce qui peut conduire à des performances limitées ou inappropriées du système. En particulier, ces perturbations peuvent par exemple gêner le pilotage d'un dispositif émetteur et/ou récepteur d'ondes et/ou entraîner la réception de données en retour dégradées, pouvant alors conduire à une qualité d'image dégradée dans des applications d'imagerie (par exemple d'imagerie ultrasonore).

### Exposé de la divulgation

L'un des objets de la présente divulgation est de résoudre au moins l'un des problèmes ou déficiences décrits précédemment.

En particulier, il peut être souhaitable d'améliorer le pilotage d'un dispositif émetteur et/ou récepteur d'ondes, notamment mais pas exclusivement d'un dispositif transducteur d'ultrasons, et/ou d'améliorer la qualité des signaux d'un tel dispositif émetteur et/ou récepteur d'ondes.

En particulier, comme décrit plus en détail ultérieurement, du bruit électrique (pouvant comprendre notamment du bruit électronique) est susceptible de perturber les signaux transmis dans des liaisons électriques utilisées pour piloter un dispositif émetteur et/ou récepteur d'ondes. Il peut ainsi être souhaitable de limiter les effets induits par un tel bruit électrique, afin notamment de limiter les perturbations pouvant affecter les signaux électriques transmis entre un système de commande et un dispositif émetteur et/ou récepteur d'ondes.

Par exemple, dans le cas où le dispositif émetteur et/ou récepteur est (ou comprend) une sonde à ultrasons, il peut être souhaitable d'améliorer les signaux électriques transmis au travers de liaisons électriques en limitant le bruit électrique susceptible de perturber cette transmission, par exemple en vue d'améliorer la qualité d'une image ultrasonore.

A cet effet, selon un premier aspect, la présente divulgation vise un procédé pour piloter un dispositif émetteur et/ou récepteur d'ondes. Le procédé selon le premier aspect comprend le pilotage du premier dispositif au travers de canaux établis via des liaisons électriques dans lequel au moins deux desdits canaux sont permutés au cours du temps parmi les liaisons électriques.

En fournissant un tel procédé, il devient avantageusement possible d'améliorer le pilotage du dispositif émetteur et/ou récepteur d'ondes et donc les performances du système dans son ensemble, notamment en lissant, ou moyennant, ou redistribuant (dans le temps et/ou l'espace), le bruit électrique susceptible de se produire parmi les liaisons électriques utilisées pour transmettre les signaux électriques, de façon à limiter les éventuelles perturbations pouvant résulter d'un tel bruit électrique. La qualité des images construites sur la base des signaux émis et reçus en retour se trouve ainsi améliorée sensiblement par exemple, dans le cadre d'une application d'imagerie ultrasonore.

Le procédé peut être mis en oeuvre avec un minimum d'adaptation dans les systèmes de pilotage existants, en implémentant de façon logicielle et/ou matérielle les moyens nécessaires pour réaliser la ou les permutations de canaux. Les coûts de mise en oeuvre du procédé peuvent donc être réduits.

Il n'est ainsi par exemple parfois plus nécessaire de mettre en oeuvre d'algorithme de correction pour limiter les défauts se produisant sur l'image en raison du bruit électrique, ce qui permet de faciliter le traitement des signaux et limiter les coûts de traitement associés en termes notamment de temps et de ressources.

Le procédé selon la divulgation peut comporter d'autres caractéristiques qui peuvent être prises séparément ou en combinaison, notamment parmi les modes de réalisation qui suivent.

Selon un exemple de réalisation, le pilotage comprend :
- établissement des canaux par un dispositif de pilotage.

Selon un exemple de réalisation, le pilotage comprend en outre :
- transmission de signaux électriques au travers des canaux.

Selon un exemple de réalisation, lesdits canaux sont des canaux analogiques pour la transmission et/ou réception de signaux électriques entre le dispositif de commande et le premier dispositif.

Selon un exemple de réalisation, le procédé comprend au moins une permutation au cours du temps desdits au moins deux canaux parmi les liaisons électriques.

Selon un exemple de réalisation, la permutation comprend une pluralité de permutations au cours du temps des canaux parmi les liaisons électriques.

Selon un exemple de réalisation, les canaux sont permutés au cours de la permutation de sorte qu'une seule liaison électrique est utilisée à chaque instant par canal.

Selon un exemple de réalisation, la permutation est réalisée selon une séquence prédéterminée au cours du temps.

Selon un exemple de réalisation, la permutation est réalisée selon une séquence aléatoire au cours du temps.

Selon un exemple de réalisation, les liaisons électriques sont intercalées entre un premier multiplexeur et un deuxième multiplexeur, chaque permutation étant causée par un changement du routage par les premier et deuxième multiplexeurs des canaux parmi les liaisons électriques.

Selon un exemple de réalisation, les premier et deuxième multiplexeurs réalisent un multiplexage des canaux entre une pluralité de pulseurs et une pluralité d'éléments piézoélectriques, chaque canal couplant un même pulseur à un même élément piézoélectrique indépendamment de la permutation.

Selon un exemple de réalisation, les liaisons électriques comprennent au moins un multiplexeur intermédiaire divisant lesdites liaisons électriques en tronçons montés en série,
dans lequel le procédé comprend au moins une dite permutation qui permute les canaux parmi les tronçons des liaisons électriques.

Selon un exemple de réalisation, le pilotage comprend :
- envoi de groupes de signaux électriques au premier dispositif au travers des canaux, chaque groupe correspondant à un tir configuré pour causer l'émission d'une onde par le premier dispositif ;
dans lequel lesdits au moins deux canaux sont permutés tous les X tirs parmi les liaisons électriques, X étant un entier supérieur ou égal à 1 (voire supérieur ou égal à 2).

Selon un exemple de réalisation, lesdites ondes sont des ondes acoustiques.

Selon un exemple de réalisation, ledit procédé est appliqué à l'imagerie médicale par ultrasons.

Selon un deuxième aspect, la présente divulgation se rapporte à un programme d'ordinateur comprenant des instructions qui, lorsque le programme est exécuté par un ordinateur, conduisent celui-ci à mettre en oeuvre le procédé selon le premier aspect. Autrement dit, les différentes étapes du procédé selon le premier aspect sont déterminées par des instructions de programmes d'ordinateurs.

Un tel programme d'ordinateur peut utiliser n'importe quel langage de programmation ou équivalent, et il peut se trouver sous la forme d'un code source, d'un code objet, ou d'un code intermédiaire entre un code source et un code objet, tel que dans une forme partiellement compilée, ou sous n'importe quelle autre forme souhaitable.

Selon un troisième aspect, la présente invention concerne un support d'enregistrement (ou support d'informations), lisible par un ordinateur (ou un processeur), sur lequel est enregistré un programme d'ordinateur comprenant des instructions pour l'exécution des étapes du procédé selon le premier aspect de la présente divulgation.

D'une part, le support d'enregistrement peut être n'importe quel entité ou dispositif capable de stocker le programme, telle qu'au moins une mémoire volatile et/ou non volatile. Par exemple, le support peut comporter un moyen de stockage, tel qu'une mémoire non-volatile réinscriptible, une mémoire ROM, un CD-ROM ou une mémoire ROM de type circuit microélectronique, ou encore un moyen d'enregistrement magnétique ou un disque dur. Cette mémoire peut par exemple comprendre une mémoire de carte graphique (ou carte vidéo), ce type de mémoire étant notamment apte à traiter des données d'image (ou données vidéo).

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon la présente divulgation peut être en particulier téléchargé grâce à un réseau filaire ou non, de type local (Bluetooth^{®} par exemple, Wi-Fi, Ethernet, Internet, 4G, 5G ou autres).

Alternativement, le support d'enregistrement peut être un circuit intégré dans lequel le programme d'ordinateur est incorporé, le circuit intégré étant adapté pour exécuter ou pour être utilisé dans l'exécution du procédé en question.

Selon un quatrième aspect, la présente divulgation concerne un système de commande pour piloter un premier dispositif émetteur et/ou récepteur d'ondes, ledit système comprenant :
- un dispositif de commande configuré pour piloter le premier dispositif au travers de canaux établis via des liaisons électriques ; et
- un dispositif de pilotage configuré pour permuter au moins deux desdits canaux au cours du temps parmi les liaisons électriques.

Selon un exemple, le système de commande comprend une mémoire associée à un processeur configuré pour la mise en oeuvre des étapes du procédé selon le premier aspect de la présente divulgation.

Le système de commande peut avoir des fonctionnalités qui correspondent aux opérations du procédé selon le premier aspect de la divulgation. En particulier, les différents modes de réalisation mentionnés ci-avant en relation avec le procédé selon le premier aspect de la divulgation ainsi que les avantages associés peuvent s'appliquer de façon analogue au système de commande selon le quatrième aspect de l'invention.

Les caractéristiques et avantages de la divulgation apparaitront plus précisément à la lecture de la description qui va suivre, donnée uniquement à titre d'exemple non limitatif, et faite en référence aux figures annexées. Notamment, les exemples illustrés dans les figures peuvent être combinés entre eux, sauf incohérence notoire.

### Brève description des figures

D'autres caractéristiques et avantages de la présente divulgation ressortiront de la description des exemples de réalisation non limitatifs de la présente divulgation ci-après, en référence aux figures 1 à 12 annexées, sur lesquelles :
[Fig. 1] illustre schématiquement les conséquences, sur un exemple d'image ultrasonore, de bruit électrique perturbant les signaux électriques acheminés au travers de liaisons électriques, selon un exemple particulier ;
[Fig. 2] montre un dessin schématique d'un système de pilotage d'un dispositif émetteur et/ou récepteur d'ondes, selon des exemples de réalisation de la présente divulgation ;
[Fig. 3] montre un dessin schématique d'un système d'imagerie ultrasonore selon des exemples de réalisation de la présente divulgation ;
[Fig. 4] montre un dessin schématique d'un système de pilotage d'un dispositif transducteur ultrasonore selon des exemples de réalisation de la présente divulgation ;
[Fig. 5] illustre schématiquement la permutation de canal dans le système de la figure 4, selon des exemples de réalisation de la présente divulgation ;
[Fig. 6] illustre schématiquement les signaux électriques transmis dans les canaux du système de la figure 4 selon des exemples de réalisation de la présente divulgation ;
[Fig. 7] montre un dessin schématique d'un système de pilotage d'un dispositif transducteur ultrasonore selon des exemples de la présente divulgation ;
[Fig. 8] est un diagramme illustrant schématiquement un procédé de pilotage selon des exemples de réalisation de la présente divulgation ;
[Fig. 9] est un diagramme temporel illustrant schématiquement des permutations de canal selon des exemples de réalisation de la présente divulgation ;
[Fig. 10] est un diagramme temporel illustrant schématiquement des permutations de canal selon des exemples de réalisation de la présente divulgation ;
[Fig. 11] est un diagramme temporel illustrant schématiquement des permutations de canal selon des exemples de réalisation de la présente divulgation ;
[Fig. 12] montre un dessin schématique d'un système de pilotage d'un dispositif transducteur ultrasonore selon des exemples de réalisation de la présente divulgation.

### Description des modes de réalisation

Comme déjà indiqué, la présente divulgation porte sur le pilotage de dispositifs émetteur et récepteur d'ondes et des procédés de pilotage de tels dispositifs. Un tel dispositif peut constituer un dispositif médical. Plus particulièrement, il peut faire partie d'un système d'échographie.

Un dispositif émetteur et/ou récepteur d'ondes peut être piloté au moyen de signaux électriques, ces signaux étant par exemple transmis entre le dispositif émetteur et/ou récepteur d'ondes et une unité de commande d'un système de commande. Ces signaux électriques peuvent être transmis ou échangés entre l'unité de commande et le dispositif émetteur et/ou émetteur d'ondes (tel qu'un dispositif transducteur par exemple) au travers de liaisons électriques. Chaque élément piézoélectrique (ou élément transducteur) d'un dispositif transducteur peut par exemple être piloté par des signaux électriques transmis via une liaison électrique dédiée. Or, il a été observé qu'un bruit électrique (pouvant comprendre notamment du bruit électronique) est susceptible de venir perturber les signaux électriques circulant dans ces liaisons électriques, en raison notamment de la dissymétrie de ces liaisons électriques les unes par rapport aux autres. Ce bruit électrique (dit aussi par la suite bruit perturbateur, ou bruit) dégrade les signaux échangés et gêne le pilotage du dispositif émetteur et/ou récepteur d'ondes, ce qui peut notamment causer une dégradation de la qualité de l'image dans des applications d'imagerie (par exemple d'imagerie ultrasonore).

En effet, les liaisons électriques utilisées pour transmettre les signaux électriques peuvent par exemple comprendre des pistes électriques formées sur une carte électronique et/ou des câbles électriques (par exemple de type coaxial). Or, des disparités physiques existent généralement entre plusieurs pistes ou liaisons électriques, notamment en termes de longueur, dimensions, conductivité électrique, impédance, etc. Quand bien même des pistes électriques seraient conçues pour être identiques ou symétriques, l'existence de telles différences physiques ou structurelles est inhérente à la réalisation de pistes ou liaisons électriques en pratique. Il a été observé que ces dissymétries entre les liaisons électriques peuvent être l'une des origines du bruit perturbateur.

Ce problème de bruit est en outre amplifié par le nombre croissant de liaisons électriques ménagées aujourd'hui dans les systèmes de pilotage (en particulier au niveau des cartes électroniques), ce qui accroit les contraintes sur le routage ou agencement de telles liaisons électriques, par exemple dans les cartes électroniques des systèmes de pilotage. Il est ainsi de plus en plus difficile d'adapter la conception de pistes électriques sur une carte de pilotage en raison des contraintes de conception (taille limitée de la carte, nombre croissant de pistes électriques, complexité croissante de la carte, etc.).

Le nombre de liaisons électriques nécessaire pour piloter un dispositif transducteur piézoélectrique est en particulier fonction du nombre d'éléments piézoélectriques présents dans le dispositif transducteur piézoélectrique. Plus le nombre d'éléments piézoélectriques est important, plus le nombre nécessaire de liaisons électriques croît. Le développement d'applications nécessitant un grand nombre d'éléments piézoélectriques (par exemple des sondes matricielles, sondes 3D, etc.) conduit à amplifier les problèmes de dissymétries et de contraintes d'agencement décrits ci-avant.

Il a été mis en évidence que le bruit électrique résulte notamment de phénomènes physiques tels que les différences de longueur de piste (« trace length mismatch » en anglais), des différences d'impédance (« impédance mismatch » en anglais), des décalages de temps de propagation (« propagationtime mismatch » en anglais), des phénomènes parasites de couplage entre les liaisons électriques (dit « crosstalk » en anglais), etc.

Par ailleurs, il a été constaté que les disparités en termes de caractéristiques ou de comportement entre les composants électroniques utilisés dans un système de pilotage, notamment dans une carte électronique, pour piloter un dispositif émetteur et/ou récepteur d'ondes peuvent également être sources de bruit(s) électrique(s) et donc de dégradation des signaux.

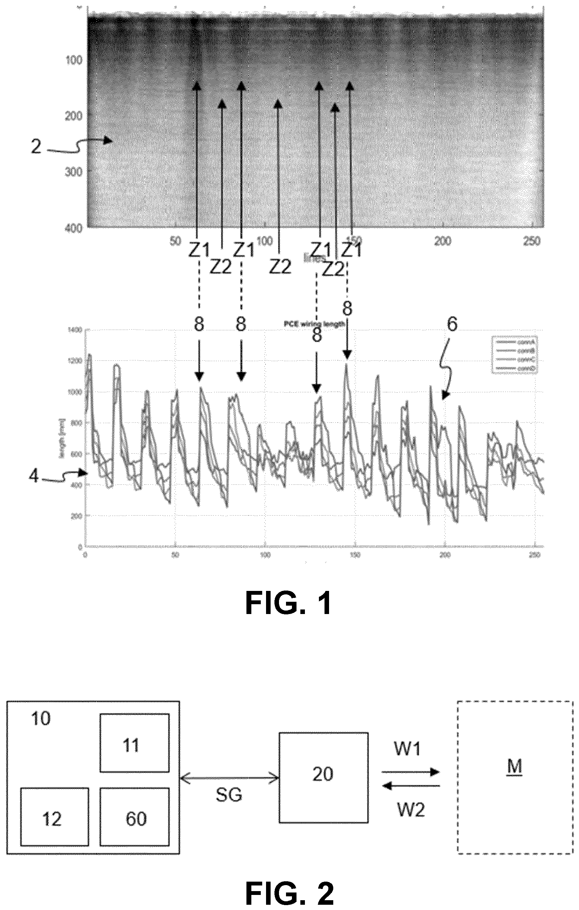

La figure 1 illustre selon un exemple particulier une image ultrasonore 2 définie par des niveaux de gris qui sont fonction de la réponse d'un milieu à des ondes ultrasonores. Dans cette image, l'axe des abscisses représente la position (ou index) des éléments piézoélectriques utilisés et l'axe des ordonnées représente la profondeur dans le milieu. On suppose ici à titre d'exemple que l'on utilise un dispositif transducteur d'ultrasons (non représenté) comprenant 256 éléments transducteurs pour sonder le milieu en question.

Il a été constaté une dégradation de la qualité de l'image ultrasonore 2 sous l'effet d'un bruit électrique présent au niveau des liaisons électriques entre un système de pilotage (non représenté) et le dispositif transducteur d'ultrasons. Cette dégradation qui conduit à une qualité d'image moindre voire très détériorée se traduit notamment par une alternance de zones sombres Z1 et de zones claires Z2 qui n'est aucunement représentative du milieu étudié. Les zones sombres Z1 de l'image 2 correspondent à des canaux qui reçoivent un signal de très faible niveau. Les zones claires Z2 sont des zones plus claires (grises ou blanches) correspondant, dans l'image 2, à des signaux de plus fort niveau, mais qui sont perturbées par du bruit de mesure qui se traduit par du bruit visuel (et non des éléments réels du milieu observé).

La figure 1 illustre également un graphe 4 comportant quatre courbes 6 représentant chacune, pour une sonde donnée, la longueur des pistes électriques (sur la carte électronique) utilisées respectivement pour piloter les éléments piézoélectriques du dispositif transducteur. Ainsi, les abscisses du graphe 4 représente la position (ou index) des éléments piézoélectriques utilisés tandis que les ordonnées représentent les longueurs des pistes électriques (en millimètres dans cet exemple).

La figure 1 montre l'effet des disparités de longueur entre les pistes électriques sur le niveau des signaux électriques reçus par les éléments piézo-électriques de la sonde. Comme illustré, ces courbes 6 forment une succession de pics 8 correspondant à des maximums de niveau de bruit électrique générés dans les liaisons électriques. Comme cela apparaît des courbes 6, le niveau du bruit électrique varie en fonction des liaisons électriques utilisées, chaque pic d'atténuation 8 correspondant dans cet exemple à une zone sombre Z1 dans l'image 2. Plus la piste électrique utilisée est longue, plus l'atténuation (ou perte) électrique est importante, et donc plus le signal électrique reçu est faible (et donc plus la région correspondante de l'image 2 est sombre).

Des algorithmes de correction peuvent être utilisés pour compenser les dégradations d'image résultant d'un tel bruit électrique mais ces algorithmes ne sont pas toujours suffisamment performants, ils ne fournissent pas toujours des résultats satisfaisants et complexifient le traitement des signaux et donc augmentent les coûts de traitement en termes de temps et de ressource, ce qui est peu compatible en particulier avec une utilisation en temps réel du système.

Un procédé et un dispositif pour piloter un dispositif émetteur et/ou récepteur d'ondes vont maintenant être décrits dans ce qui va suivre en référence conjointement aux figures 1-12 fournies à titre d'illustration. Sauf indications contraires, les éléments communs ou analogues à plusieurs figures portent les mêmes signes de référence et présentent des caractéristiques identiques ou analogues, de sorte que ces éléments communs ne sont généralement pas à nouveau décrits par souci de simplicité.

Les termes « premier(s) » (ou première(s)), « deuxième(s) », etc.) sont utilisés dans ce document par convention arbitraire pour permettre d'identifier et de distinguer différents éléments (tels que des opérations, des dispositifs, etc.) mis en oeuvre dans les modes de réalisation décrits ci-après.

Comme précédemment indiqué, l'invention vise notamment un procédé de pilotage d'un dispositif émetteur et/ou récepteur d'ondes. La figure 1 représente schématiquement un système (ou dispositif) 10, dit aussi système de commande ou dispositif de commande, configuré pour piloter un dispositif 20 émetteur et/ou récepteur d'ondes selon des exemples de réalisation de la présente divulgation.

Le dispositif 20 est configuré pour émettre et/ou recevoir des ondes W. La nature de ces ondes dépend de la configuration du dispositif 20, au vu notamment de l'utilisation qui en est faite. Selon un exemple, le dispositif 20 est configuré pour émettre des ondes W. Selon un autre exemple, le dispositif 20 est configuré pour recevoir des ondes W. Selon un autre exemple, le dispositif 20 est configuré pour à la fois émettre et recevoir des ondes W.

Les ondes W peuvent par exemple être (ou comprendre) des ondes acoustiques, par exemple de type ultrasonore. À titre d'exemple, on considère par la suite que le dispositif 20 est un dispositif transducteur à ultrasons (ou sonde à ultrasons) qui permet d'émettre et/ou recevoir des ondes ultrasonores. À noter toutefois que d'autres exemples de dispositif émetteurs et/ou récepteurs d'ondes sont possibles selon la présente divulgation comme précisé ultérieurement.

Le système de commande 10 peut comprendre dans cet exemple une unité (ou dispositif) de commande 12 (dite aussi unité de commande de puissance) configurée pour piloter le dispositif transducteur 20 au moyen de signaux électriques SG qui sont échangés (ou transmis) entre le système 10 et le dispositif transducteur 20.

Plus particulièrement, l'unité de commande 12 est configurée pour générer des signaux électriques SG qui sont transmis au dispositif transducteur 20 pour causer l'émission, par ledit dispositif transducteur 20, d'ondes ultrasonores W1 en direction et/ou dans un milieu M. Les signaux électriques SG ainsi générés sont représentatifs des (ou définissent les) ondes ultrasonores W1 projetées dans le milieu M. À cet fin, l'unité de commande 12 peut par exemple être ou comprendre au moins un pulseur électronique ou simplement « pulseur » (c'est à dire un générateur de signaux électriques). Les signaux électriques SG peuvent être de diverses formes, par exemple de forme carrée, sinusoïdale, quelconque, etc.

L'unité de commande 12 peut par exemple comprendre des dispositifs récepteurs ou circuits récepteurs (non représentés) configurés pour recevoir des signaux électriques SG en provenance du dispositif transducteur 20.

Comme illustré en figure 1, le système 10 peut également comprendre dans cet exemple une unité (ou dispositif) de traitement 11 configurée pour commander l'unité de commande 10, par exemple en commandant les signaux électriques SG. Cette unité de traitement 11 peut par exemple être ou comprendre au moins un processeur.

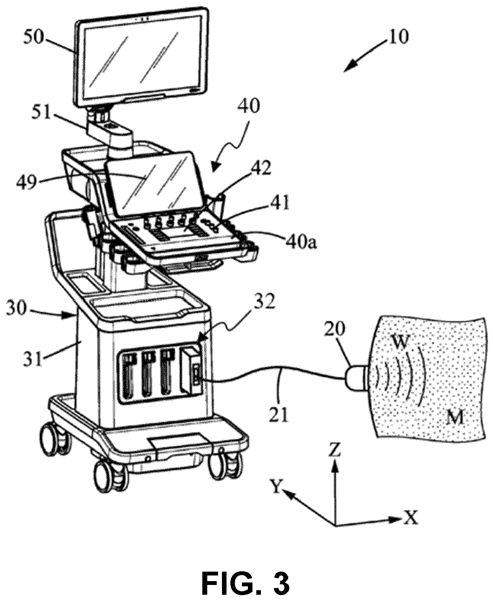

Selon un exemple, l'unité de traitement 11 et/ou l'unité de commande 12 sont comprises dans le corps 31 (élément central) du système 10 représenté en figure 3.

Plus particulièrement, l'unité de traitement 11 peut être configurée pour contrôler les signaux électriques SG qui sont générés par l'unité de commande 12. L'unité de traitement 11 peut également être configurée pour traiter (ou interpréter) des signaux électriques SG reçus par l'unité de commande 12 en provenance du dispositif transducteur 20. Ces signaux SG sont représentatifs d'ondes W2 reçues par le dispositif transducteur 20 en provenance du milieu M. Ces ondes W2 forment par exemple un ou plusieurs échos ultrasonores, c'est-à-dire une réponse du milieu M aux ondes ultrasonores W1. Le traitement réalisé par l'unité de traitement 11 sur les signaux SG reçus peut varier selon le cas et comprendre par exemple au moins l'une quelconque parmi des opérations d'amplification, de filtrage, de numérisation et de conditionnement des signaux SG.

Le système 10 peut comprendre le dispositif transducteur 20. En variante, le dispositif transducteur 20 peut être externe au système 10. Par exemple, le dispositif transducteur 20 peut être connecté au système 10 par un câble ou peut communiquer sans fil avec lui. Dans ce dernier cas, le dispositif transducteur 10 peut par exemple comprendre une batterie et recevoir des signaux de communication du système 10 qui représentent les signaux électriques SG (par exemple les fréquences de pilotage et/ou toute information comprise dans les signaux électriques). Le dispositif transducteur 20 peut alors reproduire les signaux électriques SG en interne à partir des signaux de communication reçus.

Le dispositif transducteur 20 peut par exemple être un dispositif émetteur et/ou récepteur d'ondes conventionnel. Ainsi, une différence selon la présente divulgation peut résider dans la manière dont le dispositif transducteur 20 est piloté et les moyens associés qui sont mis en oeuvre pour réaliser un tel pilotage.

Le système 10 peut être stationnaire ou mobile. Le dispositif transducteur 20 peut également être stationnaire ou mobile. Par exemple, le système 10 peut être un système fixe (par exemple comprenant une unité de traitement et un dispositif d'affichage, comme décrit ci-dessous) et le dispositif transducteur 20 peut être mobile (par exemple un dispositif capteur, un dispositif de mesure, ou une sonde). Toutefois, il est également possible que le dispositif transducteur 20 soit intégré au système 10, et que le système 10 soit un système mobile. Par exemple, le système 10 peut être configuré pour être piloté de manière autonome, par exemple grâce à une batterie incluse. D'autres exemples sont décrits ci-dessous.

Comme illustré en figure 1, le système 10 comprend en outre un dispositif de pilotage (ou unité de pilotage) 60 configuré pour piloter le dispositif transducteur 20. La configuration et le fonctionnement du dispositif de pilotage 60 apparaitront plus en détail dans les exemples de réalisation décrits ci-après.

Selon un exemple, le système 10 peut comprendre au moins une mémoire (non représentée) utilisée par l'unité de traitement 11 pour commander l'unité de commande 10. Cette mémoire peut éventuellement faire partie de l'unité de traitement 11. Dans des exemples, le processeur et la mémoire de l'unité de traitement 11 peuvent être incorporés dans le système 10 illustré en figure 3 ou peuvent être incorporés dans un ordinateur ou un dispositif informatique lié de manière communicante à celui-ci. La mémoire peut stocker des instructions pour d'exécuter les (ou au moins un des) procédés décrits dans le présent document. Cette mémoire peut notamment stoker des instructions pour traiter des données ultrasonores et/ou des instructions pour construire des images illustratives du milieu observé.

Selon la configuration et le type de dispositif informatique considéré, la mémoire de l'unité de traitement 11 peut être volatile (telle que la RAM), non volatile (telle que de la mémoire ROM, flash, EEPROM, etc. ou tout autre dispositif de stockage et/ou support lisible par ordinateur comme décrit ci-après) ou une combinaison des deux. La mémoire utilisée par l'unité de traitement 11 peut par exemple comprendre tout ou partie d'une mémoire de carte graphique (ou carte vidéo), ce type de mémoire étant notamment apte à traiter et/ou envoyer des données d'image pouvant être utilisées pour afficher une ou des images sur un écran (ou une unité) d'affichage.

Le système 10 (notamment l'unité de traitement 11) peut comprendre des dispositifs de stockage (amovibles et/ou non amovibles), y compris, mais sans s'y limiter, des disques magnétiques ou optiques ou des bandes (faisant éventuellement partie de l'unité de traitement 11).

En outre, le système 10 (notamment l'unité de traitement 11) peut comporter un ou plusieurs dispositifs d'entrée tels qu'un clavier, une souris, un stylo, une entrée vocale, etc. et/ou un ou plusieurs dispositifs de sortie tels qu'un ou des écrans, des haut-parleurs, une imprimante, etc. L'environnement peut également comprendre une ou plusieurs connexions de communication, telles que des connexions de type LAN, WAN, point à point, etc. Dans certains modes de réalisation, les connexions peuvent être utilisées pour établir des communications point à point, des communications filaires, des communications sans fil, etc.

Le système 10 (notamment l'unité de traitement 11) peut en outre comprendre au moins une certaine forme de support lisible par ordinateur. Les supports lisibles par ordinateur peuvent être n'importe quel support disponible auquel l'unité de traitement 11 (notamment son ou ses processeurs) ou d'autres dispositifs comprenant l'environnement d'exploitation peuvent accéder. À titre d'exemple, et sans limitation, les supports lisibles par ordinateur peuvent comprendre des supports de stockage informatique et des supports de communication. Les supports de stockage informatique comprennent des supports volatils et non volatils, amovibles et non amovibles, mis en oeuvre dans tout procédé ou technologie de stockage d'informations telles que des instructions lisibles par ordinateur, des structures de données, des modules de programme ou d'autres données. Les supports de stockage informatique ne comprennent pas les supports de communication.

Les supports de communication intègrent des instructions lisibles par ordinateur, des structures de données, des modules de programme ou d'autres données dans un signal de données modulé tel qu'une onde porteuse ou un autre mécanisme de transport, et comprennent tout support de fourniture d'informations. L'expression "signal de données modulé" désigne un signal dont une ou plusieurs des caractéristiques sont fixées ou modifiées de manière à coder des informations dans le signal. À titre d'exemple, et sans limitation, les supports de communication comprennent les supports câblés tels qu'un réseau câblé ou une connexion câblée directe, et les supports sans fil tels que les supports acoustiques, RF, infrarouges, micro-ondes et autres supports sans fil. Les combinaisons de l'un quelconque des éléments ci-dessus doivent également être incluses dans le champ d'application des supports lisibles par ordinateur.

Le système 10 peut être un ordinateur unique fonctionnant dans un environnement en réseau utilisant des connexions logiques avec un ou plusieurs ordinateurs distants. L'ordinateur distant peut être une station de revue, un ordinateur personnel, un serveur, un routeur, un PC de réseau, un dispositif homologue ou un autre noeud de réseau commun, et comprend généralement plusieurs ou tous les éléments décrits ci-dessus ainsi que d'autres non mentionnés. Les connexions logiques peuvent inclure toute méthode supportée par les supports de communication disponibles.

Le système 10 peut être un système d'imagerie, par exemple dans le domaine médical. Les images générées par le système peuvent être soit analysées en temps réel, par exemple par un utilisateur, soit analysées ultérieurement et/ou dans un autre lieu que celui dans lequel se trouve le système 10.

Le système 10 peut être un système médical, par exemple un système à ultrasons. En conséquence, le dispositif 20 peut être une sonde médicale et/ou à ultrasons.

Par exemple, le système 10 peut être associé à une sonde à ultrasons 20, afin d'étudier un milieu M, notamment de collecter des données ultrasonores d'un tel milieu M. Le milieu M ainsi observé peut être de diverses natures selon le cas. Il peut s'agir par exemple de tissus vivants et/ou en particulier des tissus humains. L'observation d'un milieu M comprenant une ou des structures minérales par exemple est également possible (graviers, volcan, etc.).

La sonde à ultrasons 20 peut comprendre par exemple un ou plusieurs éléments transducteurs (non représentés sur la figure 1), chacun étant configuré pour convertir un signal électrique SG reçu du système 10 en ondes ultrasonores et/ou réciproquement. Les éléments transducteurs peuvent ainsi être configurés pour transmettre (a) des ondes (ou impulsions) W1 dans le milieu M et/ou pour recevoir (b) une pluralité de signaux ultrasonores W2 du milieu M, éventuellement en réponse à la transmission (a) des ondes W1. Un réseau de transducteurs comprenant une pluralité d'éléments transducteurs peut être utilisé. Le ou les mêmes éléments transducteurs peuvent être utilisés pour émettre des ondes (ou impulsions) et recevoir les ondes formant la réponse du milieu, ou des éléments transducteurs différents sont utilisés pour l'émission et la réception des ondes. Il peut y avoir un ou plusieurs éléments transducteurs d'émission et une pluralité d'éléments transducteurs de réception. Dans une autre variante, un seul élément transducteur peut être utilisé. Les éléments transducteurs peuvent comprendre des cristaux piézoélectriques et/ou d'autres composants qui peuvent être configurés pour générer et/ou enregistrer et/ou recevoir des signaux. Dans un souci de simplification de l'exposé, il est considéré dans les exemples de réalisation qui suivent que les éléments transducteurs sont des éléments piézoélectriques.

Divers agencements du ou des éléments transducteurs sont possibles. Par exemple, un réseau de transducteurs comprenant une pluralité d'éléments transducteurs peut être utilisé. Par exemple, on peut prévoir un réseau linéaire comprenant une pluralité (par exemple entre 100 et 300) d'éléments transducteurs juxtaposés le long d'un axe X (direction horizontale ou direction du réseau X). Dans un exemple, la matrice est adaptée pour réaliser une imagerie bidimensionnelle (2D) du milieu M, mais la matrice pourrait également être une matrice bidimensionnelle adaptée pour réaliser une imagerie 3D du milieu M. En conséquence, une matrice de transducteurs peut être utilisée. Il est cependant également possible que le système comprenne une seule ligne de transducteurs mobiles dans une sonde, de sorte que l'imagerie 3D puisse être réalisée. Il est également possible que le réseau de transducteurs comporte une ou des lignes d'éléments de transduction d'émission et une ou des lignes d'éléments de transduction de réception. Le réseau de transducteurs peut également être un réseau convexe comprenant une pluralité d'éléments transducteurs alignés le long d'une ligne courbe (par exemple dans une sonde courbe). Le ou les mêmes éléments transducteurs peuvent être utilisés pour émettre une impulsion et recevoir la réponse, ou des éléments transducteurs différents sont utilisés pour l'émission et la réception. Il peut y avoir un ou plusieurs éléments transducteurs d'émission et une pluralité d'éléments transducteurs de réception.

La sonde à ultrasons 20 peut en outre comprendre ou être associée à un dispositif de commande électronique (non représenté sur la figure 1) commandant les éléments transducteurs et acquérant des signaux SG à partir de ceux-ci. Le dispositif de commande électronique peut faire partie de la sonde. En variante, le dispositif de commande électronique peut être externe à la sonde (par exemple, faire partie de l'unité de commande de puissance 12), ou consister en plusieurs dispositifs qui font partiellement partie de la sonde et sont partiellement externes à celle-ci.

En outre, l'unité de traitement 11 peut être configurée pour recevoir des données du dispositif transducteur 20, traiter des données et/ou envoyer des données à un dispositif externe, comme par exemple, un dispositif de traitement, de stockage, d'affichage, un serveur, un ordinateur sur lequel un algorithme d'intelligence artificielle (IA) est exécuté, une station de travail dédiée, ou tout autre dispositif externe.

Toutefois, le système 10 peut être n'importe quel type de système électronique. Par exemple, le système 10 peut être un autre type de système médical qu'un système d'imagerie par ultrasons. En conséquence, le dispositif transducteur 20 peut être n'importe quel type de dispositif d'imagerie ou de capteur, utilisant d'autres ondes que les ondes ultrasonores (par exemple, des ondes ayant une longueur d'onde différente de la longueur d'onde des ultrasons et/ou des ondes n'étant pas des ondes sonores).

Selon d'autres exemples, le système 10 et/ou le dispositif transducteur 20 sont configurés à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine.

Des exemples de systèmes d'imagerie médicale comprennent un système d'imagerie par ultrasons, un système d'imagerie par rayons X (par exemple système permettant d'effectuer des mammographies) et un système IRM (Imagerie par résonance magnétique).

La figure 3 représente schématiquement des exemples de réalisation du système (ou dispositif) 10 tel que précédemment décrit en référence à la figure 1, à savoir un système d'imagerie ultrasonore dans cet exemple.

Le système d'imagerie ultrasonore 10 tel que représenté en figure 2 peut comprendre :
- une sonde à ultrasons 20 (correspondant par exemple au dispositif transducteur 20 de la figure 1),
- une unité de traitement 30 configurée pour traiter ou générer une image sur la base des signaux SG reçus par la sonde 20 (cette unité 30 correspondant par exemple à l'unité de traitement 11 de la figure 1),
- un panneau de commande 40 associé, lié ou connecté à l'unité de traitement 30, ledit panneau de commande pouvant comprendre au moins l'un parmi des boutons 41 et une tablette tactile 42, et
- un ou plusieurs écrans 50 configurés pour afficher la ou les images générées par l'unité de traitement 30.

La sonde 20 peut être connectée à l'unité de traitement 30 par tout moyen de connexion approprié tel qu'un câble 21 ou une connexion sans fil. La sonde 20 est en outre capable d'émettre des ondes ultrasonores W dans un milieu M et de recevoir des ondes ultrasonores W du milieu M, lesdites ondes ultrasonores reçues étant conséquentes ou résultant de réflexions desdites ondes ultrasonores émises sur des particules diffusantes à l'intérieur dudit milieu.

La sonde 20 peut être (ou comprendre) un réseau de transducteurs comprenant une pluralité de transducteurs (non représentés), chacun convertissant un signal électrique SG en une vibration et réciproquement. Un transducteur (dit aussi élément transducteur) est par exemple un élément piézoélectrique. Le réseau de transducteurs peut par exemple comprendre 128, 256 transducteurs ou d'avantage. Le réseau de transducteurs peut être linéaire ou incurvé et peut être disposé sur une surface extérieure du milieu M de manière à être couplé au milieu et à vibrer et à émettre ou recevoir des ondes ultrasonores W.

L'unité de traitement 30 peut comprendre des dispositifs récepteurs (non représentés), comprenant (ou étant) par exemple des circuits récepteurs, configurés pour traiter (par exemple amplifier et/ou filtrer) les signaux SG reçus de la sonde 20. Ces dispositifs récepteurs peuvent par exemple comprendre des convertisseurs pour transformer les signaux reçus en données représentant le signal (par exemple des convertisseurs analogiques-numériques (ADC) configurés pour transformer une tension en un code numérique). Les données obtenues peuvent être traitées de diverses manières ; elles peuvent notamment être stockées temporairement dans une mémoire accessible à l'unité de traitement ou traitées directement pour calculer des données traitées intermédiaires (données formées en faisceau, dites « beamformed data » en anglais). L'unité de traitement 30 peut utiliser toute méthode de traitement connue pour générer et/ou traiter une ou plusieurs images ou cartes ou données sur la base des signaux reçus de la sonde, telle que la formation de faisceaux. Les données traitées associées à une image ultrasonore peuvent être :
- une image B-mode du milieu (image en mode B) généralement affichée en niveaux de gris permettant de visualiser les organes à l'intérieur du milieu, et/ou
- une image dite Doppler, montrant les mouvements de fluides dans le milieu observé, par exemple la vitesse de mouvement et/ou l'écoulement de fluides dans le milieu (image utilisant souvent des codes couleurs), par exemple utile pour visualiser les vaisseaux sanguins et leurs activités dans le milieu, ou
- une image montrant une caractéristique mécanique du milieu (élasticité), par exemple utile pour identifier des zones de dureté différente qui peuvent se révéler être des tumeurs présentes à l'intérieur du milieu (par exemple des données d'image d'élastographie par ondes de cisaillement (ShearWave^{™} Elastography, SWE) comme décrit ci-dessous).

L'écran d'affichage 50 peut être un écran de visualisation de l'image traitée par l'unité de traitement 30. L'écran d'affichage 50 peut également afficher d'autres informations telles que les échelles utilisées dans l'image, ou des informations de configuration pour le traitement ou toute information telle que des informations d'aide ou une aide gestuelle contextuelle adaptée ou adaptable au le pavé tactile 42.

L'écran d'affichage 50 peut être articulé sur un bras de support 51 pour un meilleur positionnement face à l'utilisateur. L'écran d'affichage peut par exemple être un écran de grande taille (au moins 20 pouces) pour une meilleure visualisation des images par l'utilisateur. Un écran additionnel (non représenté) peut être présent pour montrer des images au patient ou pour tout autre usage (en salle d'intervention chirurgicale par exemple).

Le panneau de commande 40a constitue tout ou partie d'une l'interface utilisateur utilisable par un utilisateur pour interagir (commander, recevoir des informations, etc.) avec le système 10.

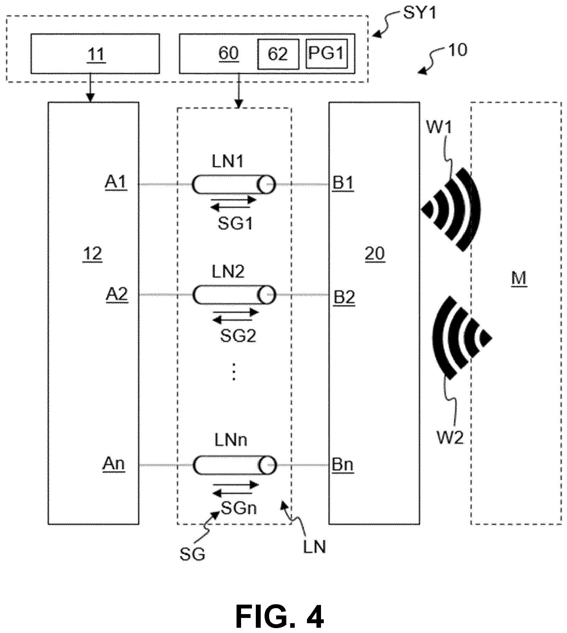

Des exemples de mise en oeuvre du système 10 selon la présente divulgation vont à présent être décrits ci-après en référence aux figures 4-12.

La figure 4 représente schématiquement des exemples de réalisation du système 10 tel que précédemment décrit en référence aux figures 1 et 2, à savoir un système d'imagerie ultrasonore dans cet exemple.

Comme indiqué, le système 10 comprend dans cet exemple une unité de commande 12 configurée pour piloter le dispositif transducteur 20 avec des signaux électrique SG. Ainsi, l'unité de commande 12 génère des signaux électriques SG qui sont envoyés au dispositif transducteur 20 pour causer l'émission d'ondes ultrasonores W1 dans le milieu M. Les signaux électriques SG ainsi générés sont représentatifs des ondes ultrasonores W1 envoyées pour exciter le milieu M. L'unité de commande 12 est également apte à recevoir des signaux électriques SG en provenance du dispositif transducteur 20 suite à excitation induite par les ondes W1 émises. Ces signaux SG sont représentatifs d'ondes W2 reçues par le dispositif transducteur 20 en provenance du milieu M. Ces ondes W2 forment par exemple un écho ultrasonore, c'est-à-dire une réponse du milieu M aux ondes ultrasonores W1.

Le système 10 comprend également une unité (ou dispositif) de traitement 11 configurée pour commander l'unité de commande 12, en particulier pour contrôler les signaux électriques SG générés par l'unité de commande 12. L'unité de traitement 11 est en outre configurée pour traiter (ou interpréter) des signaux électriques SG reçus par l'unité de commande 12 en provenance du dispositif transducteur 20.

Comme illustré en figure 4, l'unité de commande 12 utilise des liaisons électriques LN pour échanger des signaux électriques SG avec le dispositif transducteur 20, en émission et en réception. On considère par la suite qu'une pluralité de liaisons électriques notées LN1 à LNn sont ménagées entre l'unité de commande 12 et le dispositif transducteur 20, n étant un nombre entier supérieur ou égal à 2. Ce nombre n peut être adapté selon le cas. À titre d'exemple, on peut fixer n tel que n = 256.

Les liaisons électriques LN peuvent être (ou comprendre) des liaisons (ou connexions) physiques électriquement conductrices qui sont utilisées pour transporter les signaux électriques SG entre l'unité de commande 12 et le dispositif transducteur 20. La configuration de ces liaisons LN en termes notamment d'agencement et de structure peut varier selon le cas. Chaque liaison LN peut être formée par exemple en tout ou partie par au moins une piste électrique, par exemple une ou des pistes électriques formées sur une carte électronique (par exemple la carte de pilotage 32 illustrée en figure 3). Chaque liaison LN peut éventuellement comprendre au moins une portion d'un câble ou un câble électrique (par exemple de type coaxial), tel que le câble 21 illustré en figure 3. Selon un exemple, une telle liaison électrique LN peut comprendre au moins une portion de piste électrique, et éventuellement aussi au moins une portion de câble électrique.

Les liaisons électriques LN peuvent relier directement l'unité de commande 12 au dispositif transducteur 20 ou ne former qu'une partie de la connexion électrique reliant l'unité de commande 12 et le dispositif transducteur 20.

Comme illustré en figure 4, l'unité de commande 12 comprend dans cet exemple n bornes d'entrée/sortie notées respectivement A1 à An et que le dispositif transducteur 20 comprend n bornes d'entrée/sortie notées respectivement B1 à Bn. Les signaux électriques SG sont alors transmis entre l'unité de commande 12 et le dispositif transducteur 20 via les bornes A1-An et B1-Bn reliées ensemble par les liaisons électriques LN1 à LNn. Comme décrit ci-après, la manière dont ces liaisons électriques LN sont utilisées pour acheminer les signaux électriques SG entre les bornes d'entrée/sortie A1-An d'une part et B1-Bn d'autre part peut être avantageusement adaptée au cours du temps sous le contrôle du dispositif de pilotage 60.

On suppose à titre d'exemple que chaque borne B1 à Bn est associée à un élément piézoélectrique respectif (non représenté) du dispositif transducteur 20. Ainsi, chaque élément piézoélectrique est pilotable au moyen de signaux électriques SG transmis depuis une borne d'entrée/sortie A1-An de l'unité de commande 12. Des variantes sont toutefois possibles, notamment des implémentations dans lesquelles une borne B1-Bn du dispositif transducteur 20 est associée à un sous-groupe, ou un groupe, ou une pluralité, d'éléments piézoélectriques.

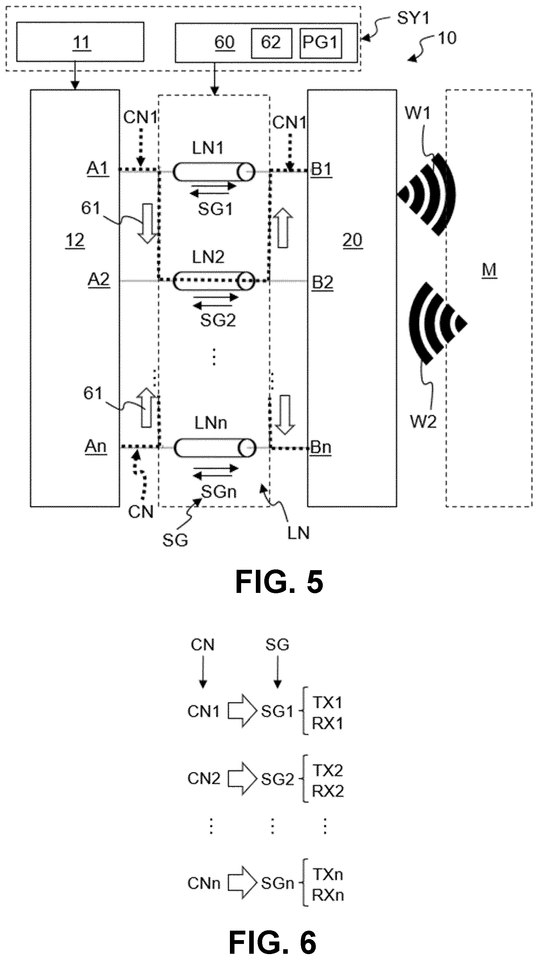

Comme illustré en figure 5, l'unité de commande 12 est configurée pour piloter le dispositif transducteur 20 (dit aussi premier dispositif) au travers de canaux CN1 à CNn (notés collectivement CN) établis via les liaisons électriques LN. Chaque canal CN forme un chemin électrique (ou parcours électrique), parmi les liaisons électrique LN, reliant (ou couplant) respectivement une borne A1-An de l'unité de commande 12 et une borne correspondante B1-Bn du dispositif transducteur 20.

Selon un exemple, les canaux CN sont des canaux analogiques.

Ceux-ci permettent la transmission et/ou la réception de signaux électriques SG entre l'unité de commande 12 et le dispositif transducteur 20 (canaux de transmission et/ou réception). Autrement dit, ces canaux CN sont configurés pour acheminer les signaux électriques SG entre l'unité de commande 12 et le dispositif transducteur 20.

Comme illustré par exemple en figure 6, chaque canal CNi (CN1 à CNn) peut être configuré pour acheminer (ou transmettre) respectivement des signaux électriques SGi (SG1 à SGn) entre l'unité de traitement 12 et le dispositif transducteur 20, i étant un nombre entier compris entre 1 et n. Comme indiqué, ces signaux SGi échangés peuvent comprendre un signal électrique TXi émis par l'unité de commande 12 et/ou un signal électrique RXi reçu par l'unité de commande 12.

Selon un exemple, les canaux CN sont des canaux filaires (ou des connexions galvaniques) dans la mesure où ils sont formés par des liaisons électriques LN de type filaire.

Le dispositif de pilotage 60 est configuré pour réaliser une ou des permutations 61 (figure 5) de canal CN au cours du temps parmi les liaisons électriques LN. Chaque permutation 61 a pour effet de modifier le parcours électrique suivi par au moins deux canaux CN parmi les liaisons électriques LN. La totalité ou seulement une partie des canaux CN peuvent avoir leur parcours modifié lors d'une même permutation.

Pour ce faire, le dispositif de pilotage 60 peut comprendre par exemple un module (ou unité) de pilotage configuré(e) pour envoyer des commandes de permutation, chaque commande de permutation déclenchant une permutation 61 d'au moins deux canaux CN parmi les liaisons électriques LN. Un tel module peut correspondre dans la présente divulgation aussi bien à un composant logiciel, qu'à un composant matériel ou à un ensemble de composants matériels et logiciels.

Comme illustré en figure 5, on suppose par exemple que le canal CN1 couplant la borne A1 de l'unité de traitement 12 à la borne B1 du dispositif transducteur 20 est initialement établi via la liaison électrique LN1 et que, sous l'effet d'une permutation 61 déclenchée par le dispositif de pilotage 60, ledit canal CN1 est reconfiguré pour s'établir via la liaison électrique LN2. Le parcours des signaux électriques SG est ainsi modifié entre les bornes A1 et B1. En parallèle, cette permutation 61 peut également conduire un autre canal CN à s'établir via la liaison électrique LN1.

On suppose par la suite pour simplification de l'illustration explicative, que chaque canal CN est configuré pour relier ensemble une même borne d'entrée/sortie A1-An de l'unité de traitement 12 à une même borne d'entrée/sortie B1-Bn du dispositif transducteur 20, et ce indépendamment de chaque permutation 61 effectuée. Plus précisément, le canal CN1 couple la paire A1/B1, le canal CN2 couple la paire A2/B2, etc. à tout instant au cours du temps. Ainsi, chaque permutation 61 de canal a pour conséquence de modifier le parcours d'au moins deux canaux CN parmi les liaisons électriques LN sans modifier les correspondances entre les bornes A1-An de l'unité de traitement 12 et les bornes B1-Bn du dispositif transducteur 20. Les permutations 61 sont donc transparentes du point de vue de l'unité de traitement 12 et du dispositif transducteur 20, ce qui permet de limiter les adaptations nécessaires au niveau du système 10 et de ne pas troubler l'utilisateur et/ou le concepteur du système.

Diverses permutations 61 de canal sont possibles dans le contexte de la présente divulgation. Des exemples de mise en oeuvre de telles permutations sont décrits ci-après.

Selon un exemple, les canaux CN peuvent être configurés de sorte qu'une seule liaison électrique LN est utilisée à chaque instant par canal CN. Autrement dit, les canaux CN sont chacun établis dans une unique liaison électrique LN, et ce indépendamment de la ou des permutations 61 effectuées. Des variantes sont toutefois possibles, par exemple des implémentations dans lesquelles les canaux CN sont établis dans plusieurs portions de différentes liaisons électriques LN, comme décrit ultérieurement.

Selon un exemple, les canaux CN peuvent être configurés de sorte qu'au moins un canal est utilisé pour piloter un élément PZ ou une pluralité d'éléments PZ (par exemple dans le cas d'une sonde matricielle).

Comme illustré en figure 5, le dispositif de pilotage 60 peut comprendre au moins un processeur 62 et une mémoire non volatile (non représentée). Le dispositif de pilotage 60, et plus généralement le système de commande 10, sont configurés pour mettre en oeuvre un procédé de pilotage pour piloter le dispositif transducteur 20 comme décrit ci-après dans des modes de réalisation particuliers. À cet effet, le dispositif de pilotage 60 peut comprendre un programme d'ordinateur PG1 stocké dans la mémoire non volatile (mémoire de type Flash ou ROM par exemple), ce programme d'ordinateur PG1 comprenant tout ou partie des instructions pour la mise en oeuvre dudit procédé. Le processeur 62 est configuré pour exécuter notamment les instructions définies par le programme d'ordinateur PG1.

La mémoire non volatile 62 peut correspondre à un quelconque dispositif de stockage (amovible et/ou non amovible) tel que celui décrit ci-avant en référence au système 10. Le dispositif de pilotage 60 peut également comprendre au moins une certaine forme de support lisible par ordinateur comme précédemment décrit en référence au système 10 (par exemple des supports de stockage informatique et/ou des supports de communication).

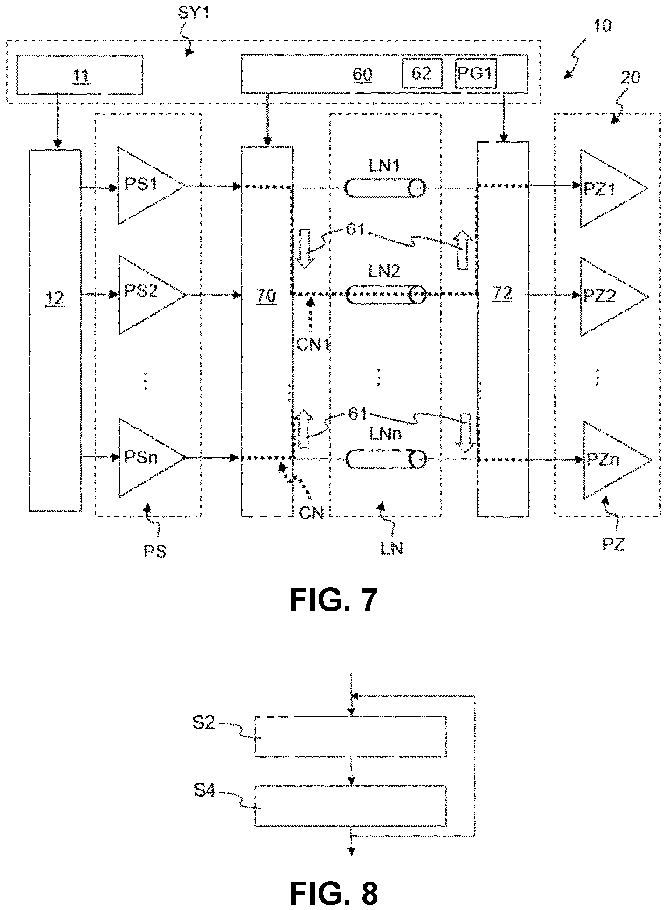

La figure 7 représente schématiquement des exemples de réalisation du système d'imagerie ultrasonore 10 décrit précédemment en référence aux figures 4-6. Comme illustré, le système 10 comprend à titre d'exemple au moins deux multiplexeurs. Ainsi, les liaisons électriques LN sont intercalées entre un premier multiplexeur 70 et un deuxième multiplexeur 72. Chaque permutation 61 de canal est causée par un changement du routage, par les premier et deuxième multiplexeurs 70 et 72, des canaux CN parmi les liaisons électriques LN.

Plus spécifiquement, les multiplexeurs 70 et 72 sont configurés pour router les signaux SG transmis (ou échangés) entre l'unité de commande 12 et le dispositif transducteur 20 parmi les liaisons électriques LN. Ce routage permet d'adapter le parcours des signaux SG parmi les liaisons électriques, et donc d'adapter les canaux CN établis via les liaisons électriques LN.

Les multiplexeurs 70 et 72 sont pilotés par le dispositif de pilotage 60 pour réaliser une ou des permutations 61 de canal au cours du temps. Chaque permutation 61 cause un changement de routage des multiplexeurs 70 et 72, conduisant à une permutation 61 d'au moins deux canaux CN parmi les liaisons électriques LN. Selon un exemple, chaque permutation 61 conduit au moins deux canaux CN à s'établir via respectivement une liaison électrique LN différente de celle précédemment utilisée directement avant ladite permutation.

Les multiplexeurs 70 et 72 peuvent être par exemple de type semiconducteur (ou « solid state » en anglais) ce qui permet notamment un montage robuste et dense dans une carte électronique.

Selon un exemple, le premier multiplexeur 70 est implémenté sous forme matérielle et/ou logicielle. Le multiplexeur 70 peut par exemple prendre la forme d'un circuit intégré, par exemple de type FPGA ( « Field-programmable gate array » en anglais). Le multiplexeur 70 peut également être implémenté au moins en partie au moyen d'un programme d'ordinateur, par exemple le programme PG1 (figures 4, 5 et 7).

Selon un exemple, le deuxième multiplexeur 72 peut être ménagé dans une sonde, telle que la sonde ultrasonore 20 (figure 3), ou dans une carte électronique, telle que la carte de pilotage 32 (figure 3). Cette dernière variante peut être avantageuse en ce qu'elle est moins complexe et permet malgré tout de limiter efficacement les effets induits par le bruit électrique comme expliqué ci-après, dans la mesure où les dissymétries de liaisons électriques LN peuvent se manifester principalement dans une telle carte de pilotage.

Comme représenté en figure 7, le système 10 peut comprendre une pluralité de pulseurs notés PS1 à PSn (notés collectivement PS) contrôlés par l'unité de commande 12 pour transmettre des signaux électriques SG au dispositif transducteur 20. Ces signaux électriques SG peuvent être ou comprendre des impulsions électroniques représentatives d'ondes W1 à émettre dans le milieu M. Les pulseurs PS peuvent faire partie de l'unité de commande 12.

Comme déjà indiqué, le dispositif transducteur 20 peut également comprendre une pluralité de transducteurs (figure 7), à savoir des éléments piézoélectriques notés PZ1 à PZn (notés collectivement PZ). Ces éléments piézoélectriques PZ sont configurés pour convertir les signaux électriques SG reçus des pulseurs PS en ondes W1 émises dans le milieu M, et pour convertir les ondes W2 reçues du milieu M en signaux électriques SG qui sont envoyés à l'unité de commande 12 pour traitement par l'unité de traitement 11. Ainsi, dans cet exemple, chaque canal CN1-CNn associe un pulseur PS1-PSn à un même élément piézoélectrique PZ1-PZn, et ce indépendamment des permutations 62 pouvant être déclenchées par l'unité de pilotage 60. Ainsi, chaque élément piézoélectrique PZ peut être piloté par un pulseur PS via un canal CN dédié.

Selon un exemple, le système 10 (par exemple l'unité de commande 12 ou l'unité de traitement 11) comprend des dispositifs récepteurs (non représentés) comme décrit ci-avant en référence notamment à la figure 3. Ces dispositifs récepteurs, comprenant (ou étant) par exemple des circuits récepteurs, sont configurés pour traiter (par exemple amplifier et/ou filtrer) des signaux SG reçus en provenance du dispositif transducteur 20. Ces dispositifs récepteurs peuvent par exemple comprendre des convertisseurs pour transformer les signaux reçus en données représentant le signal (par exemple des convertisseurs analogiques-numériques (ADC) configurés pour transformer une tension en un code numérique). Chaque dispositif récepteur est par exemple configuré pour recevoir des signaux électriques SG depuis un élément piézoélectrique PZ au travers d'un canal CN respectif.

Comme illustré, le dispositif de pilotage 60 et l'unité de traitement 11 peuvent former ensemble un système SY1, dit aussi système de pilotage. Selon un exemple, le dispositif de pilotage 60 et l'unité de traitement 11 forment un (ou font partie d'un) seul et même dispositif SY1.

Un procédé de pilotage mis en oeuvre par le système 10 tel que précédemment décrit (figures 2-7) est à présent décrit conjointement aux figures 8-11 selon des modes de réalisation particuliers. Pour ce faire, le dispositif de pilotage 60 (ou plus généralement le système 10) peut exécuter le programme d'ordinateur PG1. Le système 10 peut exécuter des instructions d'au moins un programme d'ordinateur, dont le programme PG1.

Au cours d'une étape S2 de pilotage, l'unité de commande 12 pilote le dispositif transducteur 20 au travers de canaux CN établis via les liaisons électriques LN. Les canaux CN peuvent être établis selon diverses configurations selon le cas.

Selon un exemple, le pilotage S2 comprend un établissement des canaux CN par le dispositif de pilotage 60.

Selon un exemple, le pilotage S2 comprend la transmission de signaux électrique SG au travers des canaux CN. Comme indiqué, les canaux CN peuvent être utilisés pour transmettre (ou échanger) des signaux électrique SG entre l'unité de commande 12 et le dispositif transducteur 20. Des signaux SG peuvent ainsi envoyés et/ou reçus par l'unité de commande 12 au travers des canaux CN.

Au cours d'une étape S4 de permutation, au moins deux canaux CN sont permutés 61 au cours du temps parmi les liaisons électriques LN. Dans les exemples considérés ici, la ou les permutations 61 de canal sont déclenchées par le dispositif de pilotage 60 contrôlant les multiplexeurs 70 et 72 (figure 7), par exemple par l'envoi de commandes de permutation aux multiplexeurs 70 et 72.

Ainsi, chaque permutation 61 conduit à modifier le parcours électrique d'au moins deux canaux CN parmi les liaisons électriques LN. La totalité ou seulement une partie des canaux CN peuvent avoir leur parcours modifié lors d'une permutation.

Selon un exemple, au moins une permutation 61 de canal est réalisée (S4) au cours du temps parmi les liaisons électriques LN.

Selon un exemple, une pluralité de permutations 61 de canal est réalisée (S4) au cours du temps parmi les liaisons électriques LN. Ainsi, il est possible de réaliser une succession de permutations 61 à des instants différents dans le temps pour, ces permutations modifiant à chaque fois la configuration (ou parcours électrique) des canaux CN parmi les liaisons électriques LN.

Ainsi, en permutant au moins deux canaux CN parmi les liaisons électrique LN au cours du temps, on peut avantageusement lisser ou moyenner le bruit électrique susceptible de perturber les signaux électriques SG transmis entre l'unité de commande 12 et le dispositif transducteur 20, et ainsi limiter les éventuelles perturbations pouvant résulter d'un tel bruit électrique. Les phénomènes parasites liés notamment aux dissymétries des liaisons électriques LN les unes par rapport aux autres ainsi qu'aux variations dues aux procédés de fabrication des composants et cartes électroniques, peuvent en effet être atténués ou limités en modifiant le parcours des canaux CN parmi les liaisons électriques au cours du temps. Ceci résulte d'un effet de moyennage (ou effet de lissage, ou de redistribution dans le temps et/ou l'espace) des disparités physiques ou structurelles des liaisons électriques LN causé par le changement du parcours des canaux au travers desdites liaisons électriques. Les variations des propriétés des canaux (impédances, etc.) entre les canaux peuvent notamment être réduites. Il est ainsi avantageusement possible d'améliorer les performances du système, notamment en améliorant le pilotage du dispositif émetteur et/ou récepteur d'ondes et/ou en améliorant la réponse reçue en provenance du dispositif émetteur et/ou récepteur d'ondes, et ce tout en offrant une certaine liberté de conception des liaisons électriques LN. En effet, il est possible de ménager des liaisons électriques de caractéristiques différentes (par exemple de longueurs différentes) et/ou un grand nombre de liaisons électriques, les disparités physiques ou structurelles entre ces liaisons étant compensées au moins en partie par permutation des canaux CN.

En limitant les effets induits par le bruit électrique, on peut par exemple améliorer la qualité d'image, telle que par exemple la qualité d'une image ultrasonore dans une application d'imagerie ultrasonore. Il est ainsi possible d'éviter les anomalies visuelles (par exemple une succession de zones sombres et claires) susceptibles d'être induites par le bruit électrique, comme expliqué ci-avant en référence notamment à la figure 1. Grâce au procédé de la présente divulgation, il est notamment possible d'obtenir une bonne qualité d'image sans qu'il soit nécessaire d'exécuter un algorithme de correction d'image, ce qui permet de limiter les coûts de traitement, notamment en termes de temps et de ressources, en particulier dans les cas d'applications dite en temps réel.

Selon un exemple, les canaux CN sont permutés au cours de l'étape S4 de permutation de sorte qu'une seule liaison électrique LN est utilisée à chaque instant par canal CN. Autrement dit, chaque canal CN est établi dans une unique liaison électrique LN indépendamment des permutations réalisées. Il devient ainsi possible de varier le parcours des signaux SG au travers des divers canaux CN avec un minimum de complexité. Des variantes sont toutefois possibles, notamment dans lesquelles au moins un canal CN est établi via plusieurs portions de liaison appartenant à des liaisons électriques LN différentes comme décrit ci-après.

On suppose par la suite qu'une pluralité de permutations 61 de canal est réalisée (S4) au cours du temps parmi les liaisons électriques LN. La réalisation d'une pluralité de permutations permet de garantir un bon effet de moyennage (ou redistribution, ou lissage) du bruit électrique et donc de limiter les éventuelles perturbations pouvant gêner la transmission des signaux électriques, ce qui permet notamment un meilleur pilotage du dispositif transducteur 20 et/ou une meilleure qualité d'image dans le cas d'une application d'imagerie.

Comme décrit ci-après dans des exemples illustratifs variés, des valeurs adaptées à chaque cas d'usage peuvent permettre de réaliser un compromis sur la fréquence des permutations 61 au cours du temps afin optimiser l'effet de moyennage (ou lissage, ou redistribution) du bruit électrique, dans la mesure où des permutations trop fréquentes dans le temps pourraient causer des pertes de temps, voire également perturber dans une certaine mesure les signaux électriques SG acheminés au travers des canaux CN.

Selon un exemple, les permutations sont configurées selon une fréquence de permutation qui est plus rapide que la vitesse de rafraichissement d'une image générée par le système 10, par exemple d'une image ultrasonore dans une application d'imagerie ultrasonore, de manière à ce que l'image affiche une valeur moyennée des bruits électriques. La fréquence de permutation peut par exemple être réglée pour obtenir la meilleure qualité d'image possible (peu de bruit d'imagerie, sans effet visuel gênant).

Selon un exemple, m canaux CN sont permutés lors de chaque permutation 61, m étant un nombre entier tel que 2 ≤ m ≤ n. Ce nombre m peut être fixe ou peut éventuellement varier au cours des permutations. On peut ainsi déclencher des permutations par groupe de m canaux, c'est-à-dire en causant à chaque permutation 61 une permutation de la totalité de n canaux CN ou seulement d'une sous-partie (m < n) parmi ces n canaux CN (dans ce dernier cas, au moins un canal CN n'est pas permuté).

On suppose par la suite que la totalité des n canaux CN est permutée à chaque permutation 61 bien que des variantes soient possibles où seule une sous-partie (fixe et/ou variable au cours du temps) des canaux CN permute lors des permutations 61.

Les permutations 61 au cours de l'étape S4 de permutation peuvent être mises en oeuvre de diverses manières, dont certains exemples de réalisation sont décrits ci-après.

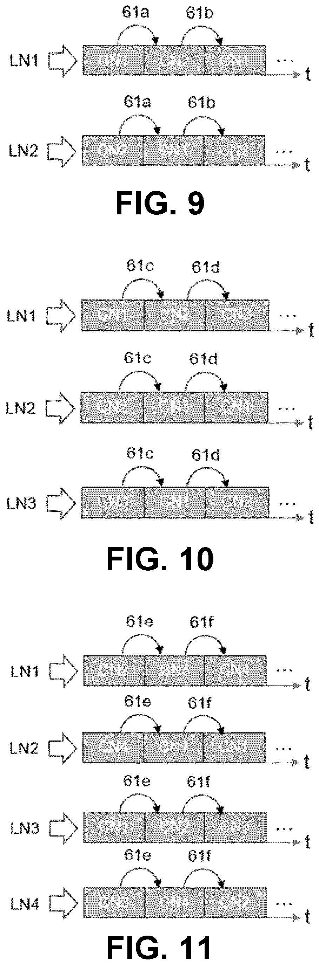

Selon un exemple, les permutations 61 sont réalisées selon une séquence prédéterminée au cours du temps. Cette séquence prédéterminée peut définir par exemple les liaisons électriques LN dans lesquelles les canaux CN sont respectivement établis à l'issue de chaque permutation 61. Dans ce cas, les permutations 61 de canal sont donc définies de façon déterministe. La séquence prédéterminée peut être indépendante des signaux électriques SG échangés entre l'unité de commande 12 et le dispositif transducteur 20 (pas d'asservissement des canaux CN en fonction des signaux acheminés). Les figures 9 et 10 décrites ci-après illustrent des exemples de pilotage basés sur des permutations déterministes.

La figure 9 est un diagramme temporel représentant schématiquement la configuration de deux canaux CN1 et CN2 au cours du temps parmi les liaisons électriques LN selon un exemple illustratif simplifié. Comme illustré dans cet exemple, deux permutations 61a et 61b sont successivement réalisées pour permuter ensemble les canaux CN1 et CN2. On considère à un stade initial que les canaux CN1 et CN2 sont respectivement établis via les liaisons électriques LN1 et LN2. La première permutation 61a conduit les canaux CN1 et CN2 à s'établir via respectivement les liaisons électriques LN2 et LN1. Le deuxième permutation 61b conduit les canaux CN1 et CN2 à revenir à leur configuration initiale en s'établissant de nouveau via respectivement les liaisons électriques LN1 et LN2. Cette séquence peut être répétées une pluralité de fois. Ainsi, les canaux CN1 et CN2 intervertissent leur chemin électrique l'une avec l'autre une pluralité de fois au cours du temps pour atteindre un effet avantageux de lissage des variations des perturbations induites comme précédemment décrit.

La figure 10 illustre un diagramme temporel représentant schématiquement la configuration de trois canaux CN1, CN2 et CN3 au cours du temps parmi les liaisons électriques LN selon un exemple. Comme illustré dans cet exemple, deux permutations 61c et 61d sont successivement réalisées pour permuter collectivement les canaux CN1-CN3. Dans cet exemple, on considère à un stade initial que les canaux CN1, CN2 et CN3 sont respectivement établis via les liaisons électriques LN1, LN2 et LN3. La première permutation 61c conduit les canaux CN1, CN2 et CN3 à s'établir via respectivement les liaisons électriques LN3, LN1 et LN2, puis la deuxième permutation 61d conduit ces canaux à s'établir via respectivement les liaisons électriques LN2, LN3 et LN1. Les permutations peuvent ainsi se répétées au cours du temps selon ce même schéma de permutations. Ainsi, les canaux CN1, CN2 et CN3 sont permutés au cours du temps de façon à effectuer une « rotation », ou permutation cyclique, dans l'occupation des liaisons électriques LN1, LN2 et LN3 permettant d'induire un effet de moyennage comme précédemment décrit.

Selon un exemple, le système 10 adapte la séquence prédéterminée des permutations 61 en fonction d'au moins un paramètre, par exemple en fonction du type de sonde utilisé (par exemple selon les plages de fréquence utilisées dans les sondes et/ou en fonction des modes d'utilisation des sondes). Par exemple, la séquence prédéterminée peut être adaptée en fonction d'un ou d'une pluralité de paramètres d'imagerie implémentés par le système 10, par exemple au moins l'un parmi : une fréquence de travail du système 10, un nombre d'éléments piézoélectrique PZ utilisés dans le système 10, un type respectif des éléments piézoélectrique PZ, un mode d'imagerie implémenté par le système 10 (tel que le « B-mode » par exemple pour générer des images en niveaux de gris).

Selon un exemple, le système 10 adapte la séquence prédéterminée des permutations 61 en fonction d'un bruit électrique présent dans les liaisons électriques LN. Ainsi, le système 10 peut par exemple analyser (ou évaluer) un bruit électrique (par exemple lors d'une phase initiale ou phase de calibration) perturbant les liaisons électriques LN et déterminer la séquence prédéterminée en fonction du bruit électrique ainsi analysé. La séquence prédéterminée des permutations 61 peut alors être construite à partir du bruit électrique estimée lors d'une phase initiale ou de calibration.

L'analyse du bruit électrique peut être réalisée de diverses manières. Cette analyse peut par exemple être à partir d'une analyse des images produites par le système 10 pour déterminer si ces images sont perturbées par du bruit électrique. L'analyse du bruit électrique peut par exemple être réalisée au moyen d'un appareil de mesure électrique (comprenant par exemple un oscilloscope) ou au moyen du système 10 lui-même. Dans ce dernier cas, les pulseurs PS peuvent alors générer des signaux électriques et les circuits de réception du système 10 peuvent être utilisés pour mesurer les signaux une fois transmis au travers des liaisons électriques LN, de façon à pouvoir évaluer l'atténuation des signaux et donc le bruit électrique affectant les liaisons électriques LN.

Selon un exemple, les permutations 61 sont réalisées selon une séquence aléatoire (ou pseudo-aléatoire) au cours du temps. Cette séquence aléatoire peut ainsi définir aléatoirement les liaisons électriques LN via lesquelles les canaux CN sont respectivement établis à l'issue de chaque permutation 61. Autrement dit, la ou les liaisons électriques LN via lesquelles sont établis chaque canal CN à l'issue d'une permutation peuvent être sélectionnées de façon aléatoire. Le caractère aléatoire des permutations 61 permet de limiter encore davantage les effets induits par le bruit électrique et notamment d'améliorer la qualité d'une image ultrasonore obtenue à partir des signaux SG traités par l'unité de traitement 11. Ceci s'explique notamment par le fonctionnement particulier de l'oeil humain qui perçoit généralement moins les dégradations d'image liées au bruit électrique lorsque les canaux sont permutés aléatoirement (effet de moyennage sur l'oeil humain). Il est en particulier possible de limiter ou éviter les périodicités des défauts (variations de luminosité, etc.) susceptibles de se produire sur une image de façon à améliorer le rendu visuel de l'image.

La figure 11 illustre un exemple de pilotage basé sur des permutations aléatoires.

La figure 11 illustre un diagramme temporel représentant schématiquement la configuration des quatre canaux CN1-CN4 au cours du temps parmi les liaisons électriques LN selon un exemple. Comme illustré dans cet exemple, deux permutations 61e et 61f sont successivement réalisées pour permuter collectivement les canaux CN1-CN4. Dans cet exemple, on considère à un stade initial que les canaux CN1, CN2, CN3 et CN4 sont respectivement établis via les liaisons électriques LN3, LN1, LN4 et LN2. Chaque permutation 61e et 61f a pour effet d'établir les canaux CN1-CN4 dans une liaison électrique LN sélectionnée aléatoirement parmi LN1-LN4. Selon un exemple, au moins un canal CN peut éventuellement être maintenu dans une même liaison électrique LN à l'issue d'une permutation tandis qu'au moins deux autres canaux CN sont permutés, bien que des variantes soient possibles où chaque canal CN change nécessairement de liaison électrique LN à chaque permutation.

Selon un exemple, le système 10 réalise des permutations 61 selon une combinaison aléatoire de séquences prédéterminées.

Selon un exemple, le système 10 réalise des permutations 61 selon au moins une séquence sélectionnée par un utilisateur parmi au moins une séquence candidate, par exemple parmi au moins une séquence prédéterminée générée par le système 10 et une séquence aléatoire. Par exemple, le système 10 peut déterminer des séquences candidates et présenter à l'utilisateur au moins une image (ou rendu visuel) générée respectivement à partir de chaque séquence candidate, l'utilisateur pouvant alors sélectionner au moins une séquence parmi les séquences candidates en s'aidant des images présentées par le système 10.

Comme précédemment décrit, les liaisons électriques LN peuvent être intercalées entre un premier multiplexeur 70 et un deuxième multiplexeur 72 (figure 7). Chaque permutation 61 peut alors être causée par un changement du routage, par les premier et deuxième multiplexeurs 70 et 72, des canaux CN parmi les liaisons électriques LN. Ces changements de routage peuvent être déclenchés par le dispositif de pilotage 60 qui commande les multiplexeurs 70 et 72. Les multiplexeurs 70 et 72 peuvent être disposés dans le système 10 de sorte à couvrir/englober des sections de liaison électrique qui sont le plus susceptibles de présenter des disparités physiques ou structurelles et donc de générer du bruit électrique.

Les multiplexeurs 70 et 72 peuvent être configurés de diverses manières. Selon un exemple, chaque canal CN peut être configuré pour relier toujours un même couple de bornes d'entrée/sortie, à savoir une borne A1-An d'une part et une borne respective B1-Bn d'autre part (figures 4-5), et ce indépendamment de la ou des permutations 61 effectuées. En outre, chaque borne d'entrée/sortie A1-An de l'unité de commande 12 peut être associée à un pulseur respectif PS1-PSn et chaque borne/d'entrée sortie B1-Bn du dispositif transducteur 20 peut être associée à un élément piézoélectrique respectif PZ1-PZn. D'autres implémentations sont toutefois possibles comme décrit ci-après.

Selon un exemple, les multiplexeurs 70 et 72 sont configurés pour réaliser au moins l'un parmi :
- un (ou au moins un) premier multiplexage des canaux CN entre la pluralité de pulseurs PS et la pluralité d'éléments piézoélectriques PZ ; et
- un (ou au moins un) deuxième multiplexage des canaux CN entre la pluralité d'éléments piézoélectriques PZ et la pluralité de dispositifs récepteurs.

Les multiplexeurs 70 et 72 peuvent ainsi réaliser un ou des premiers multiplexages et/ou un ou des deuxièmes multiplexages tels que définis ci-avant pour permuter les canaux CN utilisés respectivement pour émettre et/ou recevoir les signaux électriques SG vers/depuis le dispositif transducteur 20.

Selon un exemple, les multiplexeurs 70 et 72 réalisent au moins un premier multiplexage des canaux CN entre les pulseurs PS et les éléments piézoélectriques PZ comme décrit ci-avant. Selon une première variante, chaque canal CN (utilisé pour émettre des signaux électriques SG vers le dispositif transducteur 20) couple alors respectivement un même pulseur PS à un même élément piézoélectrique PZ indépendamment du premier multiplexage (et donc indépendamment des permutations 61). Chaque pulseur peut ainsi être couplé à un seul et même élément piézoélectrique respectif, quelles que soient les permutations réalisées. De cette manière, les permutations 61 peuvent être transparentes pour l'unité de commande 12 et le dispositif transducteur 20. Il est ainsi possible de mettre en oeuvre le procédé en limitant les adaptations de l'unité de commande 12 et du dispositif transducteur 20 et en évitant de éventuellement troubler l'utilisateur et/ou le concepteur de la machine.

Selon une deuxième variante, le premier multiplexage cause un changement de couplage par les canaux CN (utilisés pour émettre des signaux électriques SG vers le dispositif transducteur 20) entre les pulseurs PS et les éléments piézoélectriques PZ. Ainsi, un tel premier multiplexage peut conduire un élément piézoélectrique PZ à être couplé avec un dispositif récepteur différent, avant et après ledit premier multiplexage. On peut ainsi permuter les pulseurs PS en même temps que les canaux CN, c'est-à-dire modifier les pulseurs PS et les liaisons électriques LN qui sont utilisés pour piloter chaque élément piézoélectrique PZ.

Selon un exemple particulier, un élément piézoélectrique PZ peut comprendre une pluralité de sous-éléments piézoélectriques PZ.

Selon un exemple, les multiplexeurs 70 et 72 réalisent au moins un deuxième multiplexage des canaux CN entre les pulseurs PS et les dispositifs récepteurs comme décrit ci-avant. Selon une première variante, chaque canal CN (utilisé pour recevoir des signaux électriques SG depuis le dispositif transducteur 20) couple un même élément piézoélectrique PZ à un même dispositif récepteur indépendamment du deuxième multiplexage (et donc indépendamment des permutations 61). Chaque pulseur PS peut ainsi être couplé à un seul et même dispositif récepteur, quelles que soient les permutations réalisées. De cette manière, les permutations 61 peuvent être transparentes pour l'unité de commande 12 et le dispositif transducteur 20. Il est ainsi possible de mettre en oeuvre le procédé en limitant les adaptations de l'unité de commande 12 et du dispositif transducteur 20 et en évitant de éventuellement troubler l'utilisateur et/ou le concepteur de la machine.

Selon une deuxième variante, le deuxième multiplexage cause un changement de couplage par les canaux CN (utilisés pour recevoir des signaux électriques SG depuis le dispositif transducteur 20) entre les éléments piézoélectriques PZ et les dispositifs récepteurs. On peut ainsi permuter les dispositifs récepteurs en même temps que les canaux CN, c'est-à-dire modifier les dispositifs récepteurs et les liaisons électriques LN qui sont utilisés pour recevoir les signaux électriques SG des éléments piézoélectriques PZ.

Selon un exemple particulier, au cours du procédé de pilotage, l'unité de commande 12 envoie des groupes de signaux électriques SG au dispositif transducteur 20 au travers des canaux CN, chaque groupe correspondant à un tir configuré pour causer l'émission d'une (ou au moins une) onde W1 par le dispositif transducteur 20. Les canaux CN (tout ou partie d'entre eux) peuvent être permutés par exemple tous les X tirs parmi les liaisons électriques, X étant un nombre entier supérieur ou égal à 2. En effet, chaque permutation 61 de canal est susceptible de générer du bruit électrique. Pour atteindre un rapport adéquat entre la réduction du bruit électrique résultant notamment des dissymétries de ligne et du bruit secondaire pouvant résulter de ces permutations, il peut être avantageux de limiter le nombre de permutations au cours du temps, par exemple en ne déclenchant une permutation 61 que tous les X tirs (où un tir correspond par exemple à l'émission d'une onde W1). A titre d'exemple, le dispositif transducteur 20 peut être piloté via l'envoi de groupes de 128 signaux électriques (par exemple pour générer une image en niveaux de gris) ou de 1000 signaux électriques (par exemple pour générer une image couleur), chaque émission générant des signaux en retour permettant de former après traitement une image. Dans cet exemple, on peut par exemple fixer le nombre X à 2, 4 ou 10.

Il est toutefois possible de fixer X tel que X = 1. Dans ce cas, les canaux CN (tout ou partie d'entre eux) peuvent être permutés à chaque tir parmi les liaisons électriques. Plus généralement, on peut donc fixer X tel que X ≥ 1.

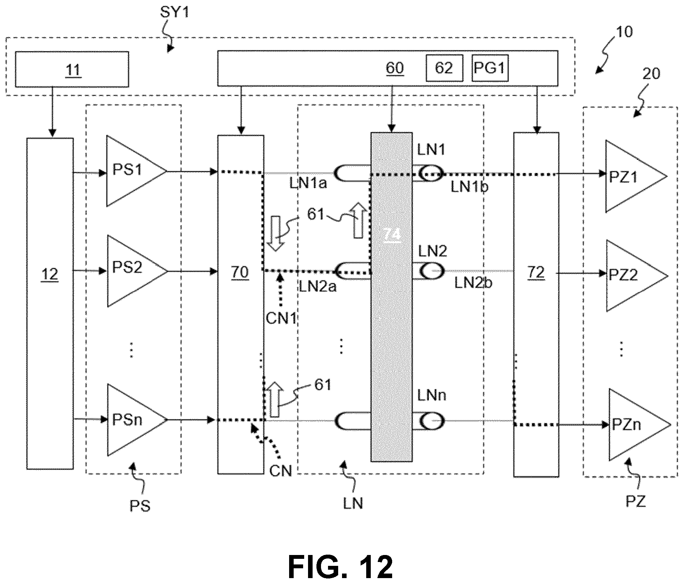

Selon un exemple, les liaisons électriques LN comprennent au moins un multiplexeur intermédiaire divisant les liaisons électriques LN en tronçons (ou portions) montés en série. Dans ce cas, le dispositif de pilotage 60 peut réaliser au moins une permutation 61 qui permute les canaux CN (tout ou partie des canaux) parmi les tronçons des liaisons électriques LN. Un mode de réalisation de cet exemple est à présent décrit en référence à la figure 12, des variantes autour de cet exemple étant toutefois possibles.

La figure 12 représente schématiquement le système 10 tel que précédemment décrit, selon un exemple de réalisation. Le système 10 se distingue de celui représenté de la figure 7 en ce qu'il comprend en outre au moins un multiplexeur intermédiaire 74. On suppose par la suite pour des raisons évidentes de simplification du propos que le système 10 comprend un unique multiplexeur intermédiaire 74 bien que cette variante soit applicable de façon analogue avec une pluralité de multiplexeurs intermédiaires 74.

Plus précisément, le multiplexeur intermédiaire 74 « divise » (ou sépare) chacune des liaisons électriques LN en deux tronçons (ou portions) montés en série. Autrement dit, l'ajout d'un étage intermédiaire de multiplexage au sein des liaisons électriques LN permet de définir des tronçons pilotables individuellement, c'est-à-dire des tronçons parmi lesquels des permutations indépendantes des canaux CN peuvent être réalisées. Ainsi, au cours de l'étape S4 (figure 8) de permutation, le dispositif de pilotage 60 peut par exemple réaliser (ou déclencher) au moins une permutation 61 qui permute les canaux CN (tout ou partie des canaux) parmi les tronçons des liaisons électriques LN. La ou les permutations 61 peuvent être réalisées par un changement du routage des canaux CN par le multiplexeur intermédiaire 74 conjointement aux multiplexeurs 70 et 72. Autrement dit, outre le contrôle des multiplexeurs 70 et 72 précédemment décrit, le dispositif de pilotage 60 contrôle le multiplexeur intermédiaire 74 pour que ce dernier change le routage des canaux parmi les tronçons des liaisons électrique LN.

À titre d'exemple, on suppose à un stade initial que le canal CN1 est établi via une première portion LN1a de la liaison électrique LN1 et une deuxième portion LN2b de la liaison électrique LN2. En réponse au contrôle du dispositif de pilotage 60, le multiplexeur intermédiaire 74 permute (ou modifie le routage) le canal CN1 pour qu'il s'établisse via une première portion LN2a de la liaison électrique LN2 et une deuxième portion LN1b de la liaison électrique LN1. Ainsi, au moins un canal CN peut être établi à un instant donné via des tronçons d'au moins deux liaisons électriques LN distinctes.

L'ajout d'au moins un étage intermédiaire de multiplexage au sein des liaisons électriques LN permet donc d'affiner le pilotage du dispositif transducteur 20 en permettant des permutations 61 de canal par tronçons, ce qui augmente les variations possibles de routage des canaux et permet d'atteindre un effet de lissage efficace y compris avec un nombre limité de liaisons électriques LN. Il est en particulier possible de cibler en priorité les changements de routage au niveau des portions de lignes électriques CN les plus exposées au bruit électrique, de manière pérenne ou selon l'utilisation de la machine, i.e ; en variant au cours du temps et/ou des applications souhaitées, afin de maximiser la réduction dudit bruit électrique.

Selon un exemple, le procédé de pilotage de la présente divulgation, tel que décrit précédemment dans des modes de réalisation, peut être appliqué à l'imagerie médicale par ultrasons. Le dispositif émetteur et/ou récepteur d'ondes 20 est (ou comprend) par exemple une sonde médicale destinée à être utilisée pour étudier/scanner (ou examiner) un milieu M, par exemple tout ou partie d'un être vivant (un patient par exemple). Pour ce faire, la sonde ultrasonore 20 peut par exemple être appliquée sur le corps d'un être vivant, éventuellement en intercalant un gel pour améliorer la transmission des ondes ultrasonores entre la sonde ultrasonore 20 et le corps en question.

Selon un exemple, chaque signal électrique SG transmis via les canaux CN est configuré pour stimuler un élément piézoélectrique PZ (figure 7) de la sonde médicale 20 et/ou représente un écho acoustique reçu par un élément piézoélectrique PZ de la sonde médicale 20.

Comme le comprend l'homme du métier, tous les modes de réalisation et variantes décrits ci-avant dont certains ont été simplifiés à dessein pour faciliter les explications, ne constituent que des exemples non limitatifs de mise en oeuvre de la présente divulgation. En particulier, l'homme du métier pourra envisager une quelconque adaptation ou combinaison des modes de réalisation et variantes décrits ci-avant, afin de répondre à un besoin particulier.

La présente invention ne se limite donc pas aux exemples de réalisation décrits ci-avant mais s'étend notamment à un procédé de pilotage qui inclurait des étapes secondaires sans pour cela sortir de la portée de la présente invention. Il en serait de même d'un dispositif de pilotage, ou plus généralement d'un système de pilotage, pour la mise en oeuvre d'un tel procédé.

## Revendications

1. Procédé pour piloter un premier dispositif (20) émetteur et/ou récepteur d'ondes (W), ledit procédé comprenant :
- pilotage (S2) du premier dispositif au travers de canaux (CN) établis via des liaisons électriques (LN) ;
dans lequel au moins deux desdits canaux sont permutés au cours du temps parmi les liaisons électriques.

2. Procédé selon la revendication 1, dans lequel le pilotage comprend :
- établissement des canaux (CN) par un dispositif de pilotage (60) ; et
- transmission de signaux électriques (SG) au travers des canaux.

3. Procédé selon la revendication 1 ou 2, dans lequel lesdits canaux (CN) sont des canaux analogiques pour la transmission et/ou réception de signaux électriques (SG) entre un dispositif de commande et le premier dispositif.

4. Procédé selon l'une quelconque des revendications précédentes, dans lequel le procédé comprend au moins une permutation (61) au cours du temps desdits au moins deux canaux parmi les liaisons électriques (LN).

5. Procédé selon la revendication 4, dans lequel la permutation comprend une pluralité de permutations (61) au cours du temps des canaux (CN) parmi les liaisons électriques.

6. Procédé selon les revendications 4 ou 5, dans lequel les canaux (CN) sont permutés au cours de la permutation (61) de sorte qu'une seule liaison électrique est utilisée à chaque instant par canal.

7. Procédé selon l'une quelconque des revendications 4 à 6, dans lequel la permutation (61) est réalisée selon une séquence prédéterminée au cours du temps.

8. Procédé selon l'une quelconque des revendications 4 à 6, dans lequel la permutation (61) est réalisée selon une séquence aléatoire au cours du temps.

9. Procédé selon l'une quelconque des revendications 4 à 8, dans lequel les liaisons électriques sont intercalées entre un premier multiplexeur (70) et un deuxième multiplexeur (72), chaque permutation (61) étant causée par un changement du routage par les premier et deuxième multiplexeurs des canaux parmi les liaisons électriques.

10. Procédé selon la revendication 9, dans lequel les premier et deuxième multiplexeurs (70, 72) réalisent au moins l'un parmi :
- un premier multiplexage des canaux (CN) entre une pluralité de pulseurs (PS) et une pluralité d'éléments piézoélectriques (PZ) ; et
- un deuxième multiplexage des canaux (CN) entre la pluralité d'éléments piézoélectriques et une pluralité de dispositifs récepteurs.

11. Procédé selon la revendication 10, dans lequel chaque canal couple un même pulseur à un même élément piézoélectrique indépendamment du premier multiplexage.

12. Procédé selon la revendication 10, dans lequel le premier multiplexage cause un changement de couplage par les canaux entre la pluralité de pulseurs (PS) et la pluralité d'éléments piézoélectriques (PZ).

13. Procédé selon l'une quelconque des revendications 10 à 12, dans lequel chaque canal couple un même élément piézoélectrique à un même dispositif récepteur indépendamment du deuxième multiplexage.

14. Procédé selon l'une quelconque des revendications 10 à 12, dans lequel le deuxième multiplexage cause un changement de couplage par les canaux entre la pluralité d'éléments piézoélectriques (PZ) et la pluralité de dispositifs récepteurs.

15. Procédé selon l'une quelconque des revendications 9 à 14, dans lequel les liaisons électriques (LN) comprennent au moins un multiplexeur intermédiaire (74) divisant lesdites liaisons électriques en tronçons (LN1a, LN1b, LN2a, LN2b) montés en série, dans lequel le procédé comprend au moins une dite permutation (61) qui permute les canaux (CN) parmi les tronçons des liaisons électriques.

16. Procédé selon l'une quelconque des revendications précédentes, dans lequel le pilotage comprend :
- envoi de groupes de signaux électriques (SG) au premier dispositif (20) au travers des canaux (CN), chaque groupe correspondant à un tir configuré pour causer l'émission d'une onde par le premier dispositif ;
dans lequel lesdits au moins deux canaux sont permutés tous les X tirs parmi les liaisons électriques, X étant un entier supérieur ou égal à 2.

17. Procédé selon l'une quelconque des revendications précédentes, dans lequel lesdites ondes sont des ondes acoustiques.

18. Procédé selon l'une quelconque des revendications précédentes, dans lequel ledit procédé est appliqué à l'imagerie médicale par ultrasons.

19. Programme d'ordinateur (PG1) comportant des instructions pour l'exécution des étapes d'un procédé selon l'une quelconque des revendications 1 à 18 lorsque ledit programme est exécuté par un ordinateur.

20. Système de commande (10) pour piloter un premier dispositif (20) émetteur et/ou récepteur d'ondes (W), ledit système comprenant :
- un dispositif de commande (12) configuré pour piloter le premier dispositif au travers de canaux (CN) établis via des liaisons électriques (LN) ; et
- un dispositif de pilotage (60) configuré pour permuter au moins deux desdits canaux au cours du temps parmi les liaisons électriques.

21. Système comprenant le système de commande (10) selon la revendication 20 et un deuxième dispositif (20) piloté par ledit dispositif de commande.
